# EUROPEAN PATENT APPLICATION

(11) **EP 4 141 016 A1**
(43) Date of publication of application: **01.03.2023**
(21) Application number: 20925653.6
(22) Date of filing: 07.04.2020
(51) Int. Cl.: C07K 1/22

(54) **TREATMENT METHOD, PRODUCTION METHOD, AND HYDROXYAPATITE FILLER**

(30) Priority: 17.03.2020 JP 2020047003
(71) Applicant: Hoya Technosurgical Corporation, Tokyo 160-0004 (JP)
(72) Inventor: KATO Machiko, Shinjukuku, Tokyo 1600004 (JP); YOSHITAKE Tomohiko, Shinjukuku, Tokyo 1600004 (JP)
(74) Representative: Diehl & Partner
(86) International application number: PCT/JP2020/015698
(87) International publication number: WO 2021/186750

(57) **Abstract**

[Problems] To provide a method for treating hydroxyapatite filler so that it can be used multiple times in the separation of a charged material included in a sample liquid using adsorbent composed of the hydroxyapatite filler, a production method including the treatment method, and hydroxyapatite filler.

[Means to solve problems] The treatment method of the present invention comprises a first step of bringing a first liquid containing a predetermined material into contact with hydroxyapatite filler, and a second step of bringing a second liquid containing an alcohol into contact with the hydroxyapatite filler.

## Description

### FIELD OF THE INVENTION

The present invention relates to a treatment method capable of improving the durability of hydroxyapatite filler, a production method including the treatment method, and hydroxyapatite filler.

### BACKGROUND OF THE INVENTION

Hydroxyapatite filler in the form of powder including secondary particles of hydroxyapatite has excellent biocompatibility, and thus has been widely used as adsorbent in a column for liquid chromatography (adsorption apparatus) by which a charged material such as a protein included in a sample liquid is adsorbed and desorbed (for example, see Patent Reference 1).

In such a column for liquid chromatography, the separation of the charged material included in the sample liquid is conducted by adsorbing the charged material on the adsorbent (hydroxyapatite filler) filled in a column body, and then desorbing it from the adsorbent. The separation (purification) of the charged material (the target material to be isolated) in the sample liquid can be carried out multiple times by repeating the adsorption and desorption.

During the repeated separation of the charged material (target material) using the adsorbent composed of the hydroxyapatite filler, and the preservation of the adsorbent by supplying a preservation liquid into the column body after the separation, high pressure is applied to the adsorbent, particularly that positioned near the outlet-side opening in the column body, into which the sample liquid is supplied. This turns a particulate shape of the adsorbent (powder) to a sand or bulk shape. As the number of the repetition increases, higher pressure is needed to supply the sample liquid into the column body. As a result, the sample liquid cannot finally be supplied into the column body. Thus, the present inventor has found that there may be room for making the adsorbent composed of the powder (including secondary particles) of hydroxyapatite usable longer to repeatedly separate the charged material (target material) included in the sample liquid.

### PATENT REFERENCE

Patent Reference 1: JP 2013-534252 A

### OBJECT OF THE INVENTION

An object of the present invention is to provide a method for treating hydroxyapatite filler so that it can be used multiple times in the separation of a charged material included in a sample liquid using adsorbent composed of the hydroxyapatite filler, a production method including the treatment method, and hydroxyapatite filler.

### SUMMARY OF THE INVENTION

The above object will be achieved by the present invention described in the following (1) to (12).
(1) A treatment method comprising a first step of bringing a first liquid containing a predetermined material into contact with hydroxyapatite filler, and
a second step of bringing a second liquid containing an alcohol into contact with the hydroxyapatite filler.

This can provide the hydroxyapatite filler with excellent durability.

(2) The treatment method described in the above (1), wherein the alcohol is a lower alcohol.

This can reliably provide the adsorbent composed of the hydroxyapatite filler with excellent durability.

(3) The treatment method described in the above (1) or (2), wherein the content of the alcohol in the second liquid is 20 % or more by weight.

This can reliably provide the adsorbent composed of the hydroxyapatite filler with excellent durability.

(4) The treatment method described in any one of the above (1) to (3), wherein the second liquid further contains a calcium ion.

This can reliably provide the adsorbent composed of the hydroxyapatite filler with excellent durability.

(5) The treatment method described in the above (4), wherein the concentration of the calcium ion in the second liquid is 1.0 mM or more and 20.0 mM or less.

This can reliably provide the adsorbent composed of the hydroxyapatite filler with excellent durability.

(6) A treatment method comprising a first step of bringing a first liquid containing a predetermined material into contact with hydroxyapatite filler, and
a second step of bringing a second liquid containing NaOH with a concentration of 0.05M or less into contact with the hydroxyapatite filler for 12 hours or more.

This can provide the hydroxyapatite filler with excellent durability.

(7) The treatment method described in any one of the above (1) to (6), wherein the second step, in which the second liquid is brought into contact with the hydroxyapatite filler, has a process of passing the second liquid through the hydroxyapatite filler in an amount two times or more as much as that of the latter, and then keeping the hydroxyapatite filler in the second liquid.

This can provide stable contact of the adsorbent with the treatment liquid.

(8) The treatment method described in any one of the above (1) to (7), wherein the first step comprises a process of supplying a phosphate buffer solution containing phosphate as a predetermined material, as a first liquid, into the hydroxyapatite filler,
a recovery process comprising bringing a phosphate buffer solution containing phosphate and other salt as a predetermined material, as a first liquid, into contact with the hydroxyapatite filler,
a process of washing the hydroxyapatite filler by bringing a phosphate buffer solution containing phosphate as a predetermined material, as a first liquid, into contact with the hydroxyapatite filler,
an alkali-washing process comprising bringing an alkali solution containing alkali as a predetermined material, as a first liquid, into contact with the hydroxyapatite filler, and
a pH equilibration process comprising bringing a phosphate buffer solution containing phosphate as a predetermined material, as a first liquid, into contact with the hydroxyapatite filler.

These processes can provide the hydroxyapatite filler in contact with the first liquid containing the predetermined material.

(9) The treatment method described in any one of the above (1) to (8), wherein the first step further comprises a process of supplementing Ca by bringing the hydroxyapatite filler into contact with the first liquid containing a calcium ion, before the washing process.

These processes can prepare the hydroxyapatite filler with which the first liquid containing the predetermined material has been brought into contact.

(10) A production method comprising the steps of treating the hydroxyapatite filler by the treatment method described in any one of the above (1) to (9),
preparing a liquid composition containing a target material, and
separating the target material from the liquid composition using the hydroxyapatite filler treated in the treatment step.

The production method of the present invention can purify the target material with excellent accuracy.

(11) A production method comprising a step of preparing the hydroxyapatite filler which has already been used once or more in a step of separating a target material from a liquid composition containing it, and
a restoration step of providing the hydroxyapatite filler with improved durability by treating the hydroxyapatite filler by the treatment method described in any one of the above (1) to (9).

The production method of the present invention can provide the hydroxyapatite filler with improved durability.

(12) Hydroxyapatite filler obtained by the production method described in the above (11).

This can provide the hydroxyapatite filler with improved durability.

### EFFECTS OF THE INVENTION

The treatment method of the present invention can provide hydroxyapatite filler with excellent durability so that the separation of a charged material included in a sample can be stably carried out multiple times using the adsorbent composed of the hydroxyapatite filler.

### BRIEF DESCRIPTION OF THE DRAWINGS

Fig. 1 is a vertical cross-sectional view showing an example of an adsorption apparatus having adsorbent applicable to the treatment method and production method of the present invention.
Fig. 2 is a flow chart showing a treatment method of the hydroxyapatite filler according to the present invention.

### DESCRIPTION OF THE PREFERRED EMBODIMENTS

The treatment method, the production method and the hydroxyapatite filler of the present invention will be described in detail below on the basis of the preferred embodiment shown in the attached drawings.

Before describing the treatment method of the present invention, an example of an adsorption apparatus including adsorbent (the hydroxyapatite filler of the present invention) which can be applied to the treatment method and production method of the present invention will be described.

In the example described below, a charged material to be separated (isolated) using the adsorption apparatus is a protein, that is, the charged material includes a negatively charged material and a positively charged material, and the negatively charged material is an acidic protein and the positively charged material is a basic protein.

### <Adsorption apparatus>

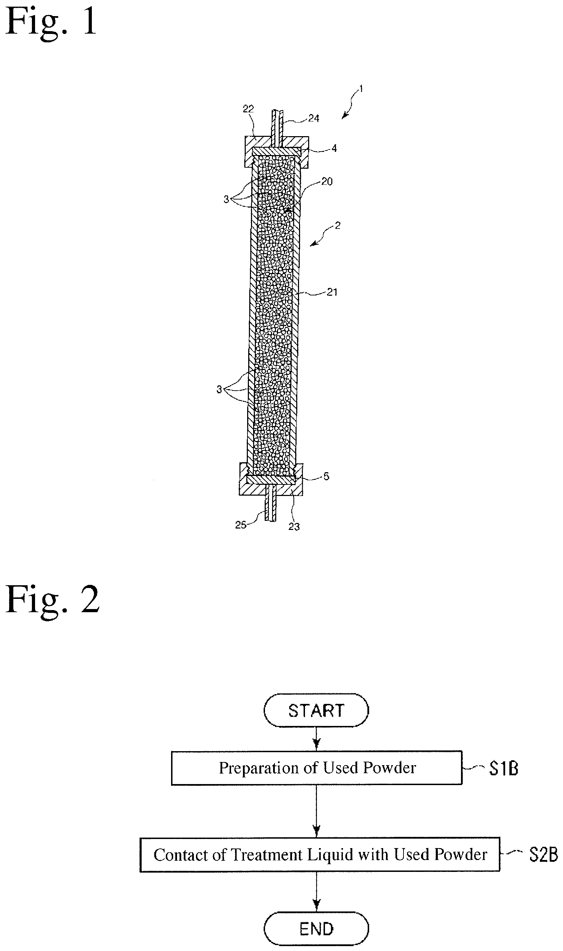

Fig. 1 is a vertical cross-sectional view showing an example of the adsorption apparatus having the adsorbent applicable to the treatment method and production method of the present invention. It is noted that the upper side in Fig. 1 is referred to as an "inlet side," and the lower side in Fig. 1 is referred to as an "outlet side," hereafter.

Herein, the inlet side means a side through which a liquid such as a sample liquid (liquid including a sample), a phosphate buffer solution (eluent), etc. is supplied into the adsorption apparatus in the separation (purification) of target protein to be isolated. On the other hand, the outlet side means a side opposite to the inlet side, through which the liquid flows out from the adsorption apparatus.

The adsorption apparatus 1 shown in Fig. 1 for separating (purifying) the protein from the sample liquid has a column 2, particulate adsorbents 3 (filler), and two filter members 4, 5.

The column 2 is constituted by a column body 21, and caps (lid bodies) 22, 23 attached to the inlet-side end and the outlet-side end of the column body 21 respectively.

The column body 21 is configured by, for example, a cylindrical member. Each component (member) of the column 2, which includes the column body 21, may be formed by, for example, various glass materials, various resin materials, various metal materials, various ceramic materials, etc.

After the filter members 4, 5 are covered on the inlet-side opening and the outlet-side opening of the column body 21 respectively, the caps 22, 23 are screwed in the inlet-side end and the outlet-side end thereof respectively.

In the column 2 of the above configuration, an adsorbent filling space 20 is defined by the column body 21 and the filter members 4, 5. The adsorbent filling space 20 is filled at least partially (fully in this embodiment) with the adsorbents 3.

The capacity of the adsorbent filling space 20 is appropriately set according to the volume of the sample liquid, without being particularly limited, but it is preferably about 0.1 mL or more and 100 mL or less, and more preferably about 1 mL or more and 50 mL or less, per 1 mL of the sample liquid.

The column body 21 equipped with the caps 22, 23 secures liquid tightness therebetween.

An inlet pipe 24 and an outlet pipe 25 are liquid-tightly fastened (fixed) to almost the centers of the caps 22, 23, respectively. The liquid is supplied to the adsorbents 3 through the inlet pipe 24 and the filter member 4. The liquid supplied to the adsorbents 3 passes through spaces (gaps) between the adsorbents 3, and then flows out of the column 2 through the filter member 5 and the outlet pipe 25. At this time, the protein to be separated (hereinafter, may be simply referred to as "protein"), and the other material which may include foreign protein other than the protein to be separated (hereinafter, may be simply referred to as "foreign protein"), in the sample liquid (sample), are separated on the basis of the difference of adsorptivity on the adsorbents 3 and the difference of affinity to a buffer solution such as the phosphate buffer solution, etc.

Each filter member 4, 5 has a function of preventing the adsorbents 3 from flowing out of the adsorbent filling space 20. Each filter member 4, 5 is preferably formed by, for example, a nonwoven fabric, a foam (sponge-like porous body with communicating holes), a woven fabric, a mesh, etc. composed of synthetic resin such as polyurethane, polyvinyl alcohol, polypropylene, polyether-polyamide block copolymer, polyethylene terephthalate, polybutylene terephthalate, etc.

The adsorbents 3 are composed of hydroxyapatite filler, specifically for example, of powder including secondary particles of hydroxyapatite (Ca₁₀(PO₄)₆(OH)₂). The protein to be separated is specifically adsorbed on the adsorbents 3 with its intrinsic adsorption (retention) power, and then separated and purified from the other material (including the foreign protein) according to the difference of their adsorption powers.

In general, proteins are classified into negatively charged acidic proteins containing a relatively large amount of acidic amino acids as their constituent amino acids, and positively charged basic proteins containing a relatively large amount of basic amino acids as their constituent amino acids.

On the other hand, hydroxyapatite has a positively charged Ca site and a negatively charged phosphate site in its crystal structure.

Accordingly, the acidic protein forms an ionic bond between its acidic amino acid and the Ca site of hydroxyapatite, and the basic protein forms an ionic bond between its basic amino acid and the phosphate site of hydroxyapatite. The binding (adsorption) strength of the protein to hydroxyapatite differs according to the amount of charge of each protein (acidic protein and basic protein).

Therefore, the adsorbents 3 composed of the hydroxyapatite filler can separate the protein (acidic protein and basic protein) and the other material (for example, the foreign protein, etc.) using the difference of their adsorption powers thereon.

Because hydroxyapatite is a calcium phosphate compound which has particularly similar components to those constituting a living body, it can suitably prevent the deterioration (denaturation) of the protein in the adsorption and separation thereof. Further, there is an advantage that the protein can be specifically removed from the adsorbents 3, for example, by providing the phosphate buffer solution (eluent) with gradient of the salt concentration.

At least one of the hydroxyl groups of the hydroxyapatite may be substituted with a fluorine atom. The hydroxyapatite substituted with the fluorine atom (hereinafter referred to as "fluoroapatite") can more reliably prevent a calcium atom (calcium ion) from being detached therefrom due to the presence of the fluorine atom (fluorine ion) in the crystal structure.

It is noted that the hydroxyapatite and the fluoroapatite with the substituted fluorine atom may be collectively called as "apatite," hereinafter.

The form (shape) of the adsorbents 3, which are composed of the hydroxyapatite filler as described above, is preferably particulate (granular) as shown in Fig. 1, and also may be, for example, a pellet shape (small block shape), a block shape (for example, a porous body shape whose adjacent voids communicate with each other, and a honeycomb shape), etc. Because the adsorbents 3 with a particulate shape have a large surface area, separation characteristics of the protein can be improved.

The average size of the particulate adsorbents 3 is, although not particularly limited, preferably about 0.5 µm or more and 150 µm or less, and more preferably about 10 µm or more and 80 µm or less. The adsorbents 3 with the above average size can reliably prevent the filter member 5 from clogging, as well as sufficiently secure their surface area.

The adsorbent 3 (hydroxyapatite filler) may be composed of the secondary apatite particle entirely, or of a carrier (substrate) coated with the secondary apatite particle on its surface. Among them, the adsorbent 3 entirely composed of the secondary apatite particle is preferable, thereby further enhancing the adsorption power of the adsorbent 3. As a result, the column 2 suitable for separating a large quantity of the protein can be obtained.

The adsorbents 3 entirely composed of the secondary apatite particles can be obtained, for example, by forming apatite particles (primary particles) using a wet synthesis method or a dry synthesis method, drying and granulating a slurry containing the apatite particles to obtain dried particles (secondary particles), and then sintering the dried particles.

On the other hand, the adsorbent 3 composed of a carrier coated with apatite on its surface can be obtained, for example, by colliding (hybridizing) the dried particles with the carrier made of a resin, etc.

When the adsorbent filling space 20 is fully filled with the adsorbents 3 as in this embodiment, the adsorbents 3 preferably have almost the same composition at respective portions of the adsorbent filling space 20, thereby providing the adsorption apparatus 1 with a particularly excellent ability of separating (purifying) the protein.

Further, a portion of the adsorbent filling space 20 (for example, a portion on the side of the inlet pipe 24) may be filled with the adsorbents 3, with the other portion filled with other adsorbents.

### <Separation method>

The separation method of the protein using the adsorption apparatus 1, in which powder including the secondary apatite particles is used as the adsorbents 3, will be described.

### [S1A] Preparation step (Composition-preparing step)

A sample liquid (liquid composition) containing the protein (target material) to be separated (purified) and other material (contaminant) is prepared.

When the protein is genetically modified protein (monoclonal antibody, etc.), the sample liquid may include a secretion from mammals such as sheep, rabbits, chickens, etc., animals such as insects such as silkworms, animal cells such as CHO cells derived from Chinese hamster ovarian cells, microorganisms such as Escherichia coli, etc., into which a nucleic acid including the gene coding the protein is transferred, and a cytosol component thereof, etc.

When the protein is natural protein, the sample liquid may include, for example, a blood (plasma) derived from various animals, a body fluid such as a lymph, a saliva and a nasal discharge, etc.

Among the protein, the acidic protein may be, although not particularly limited, for example, BSA, HAS, fibrinogen, pepsinogen, α-globulin, β-globulin, γ-globulin, etc. On the other hand, the basic protein may be, although not particularly limited, for example, lysozyme, thaumatin, cytochrome C, ribonuclease, trypsinogen, chymotrypsinogen, α-chymotrypsin, histone, protamine, polylysine, monellin, etc.

It is noted that the protein (acidic protein and basic protein) may be a variant obtained by replacing some amino acids of the amino acid sequence thereof with other amino acids.

As the sample liquid, the secretion from the microorganisms, etc. and the body fluid derived from animals may be used as they are, but preferable are those diluted with a neutral buffer solution such as water and physiological saline, or a phosphate buffer solution, or those filtered by a filtration membrane such as a filter. Further, the sample liquid may contain plural kinds of the protein.

As the phosphate buffer solution, the same one as in a fractionation step [S4A] described below may be used.

When the sample liquid diluted with a phosphate buffer solution is used, the salt concentration in the sample liquid is preferably about 0.5 mM or more and 30 mM or less, and more preferably about 1 mM or more and 20 mM or less.

### [S2A] Substitution step

A buffer solution is supplied into the column 2 through the inlet pipe 24, with which the inside of the column 2 is substituted.

As the buffer solution, a phosphate buffer solution is preferably used. The phosphate buffer solution may be the same one as that used as an eluent in the following step [S4A].

When a phosphate buffer solution is used as the buffer solution, the phosphate concentration in the phosphate buffer solution is preferably about 0.5 mM or more and 500 mM or less, and more preferably about 1 mM or more and 200 mM or less. With the inside of the column 2 substituted with the phosphate buffer solution having the above phosphate concentration as the buffer solution, it is possible to make the adsorbents 3 more suitable for the following separation of the protein.

It is noted that the substitution step [S2A] can be omitted according to the kinds of the protein to be separated, etc.

### [S3A] Supply step

By supplying the sample liquid obtained in the step [S1A] into the column body 21 through the inlet pipe 24 and the filter member 4, the sample liquid passes through the inside of the column 2 (adsorption apparatus 1) to be brought into contact with the adsorbents 3.

As a result, the protein to be separated (purified) having a high adsorption ability on the adsorbents 3, and some (protein) of the contaminant (foreign protein etc.) which has a relatively high adsorption ability on the adsorbents 3, are retained in the column 2. The other of the contaminant which has a low adsorption ability on the adsorbents 3 flows out from the inside of the column 2 through the filter member 5 and the outlet pipe 25.

### [S4A] Fractionation step (Collection step, separation step)

An eluent for eluting the protein such as a phosphate buffer solution is supplied into the column 2 through the inlet pipe 24. Then, an eluate flows out from the inside of the column 2 through the outlet pipe 25, and then is fractionated (collected) in predetermined amounts. That is, the protein and contaminant adsorbed on the adsorbents 3 are dissolved into the eluent according to the difference of their respective adsorption powers on the adsorbents 3, and then collected (separated and purified) into the respective fractions. Therefore, the protein (target material) is obtained by separating it from the sample liquid (liquid composition) using the adsorbents 3.

Namely, because the protein and the contaminant are specifically adsorbed on the adsorbents 3 with their respective intrinsic adsorption (retention) powers, the protein is purified by separating the protein and the contaminant according to the difference of the adsorption powers, and then selectively collecting the fraction in which the protein is dissolved.

The buffer solution used as the eluent is preferably a phosphate buffer solution. The phosphate buffer solution may be, for example, an aqueous solution containing phosphate such as sodium phosphate, potassium phosphate, lithium phosphate, ammonium phosphate, etc. Other than the phosphate buffer solution, the buffer solution may be a 2-morpholinoethanesulfonic acid (MES) or sulfuric acid.

When a phosphate buffer solution is used as the eluent, the phosphate buffer solution may further contain other salt than phosphate. The other salt may include, for example, NaCl, KCl, NH₄Cl, MgCl₂, etc.

The other salt concentration of the phosphate buffer solution is preferably 0.1 M or more and 1.5 M or less, and more preferably 0.5 M or more and 1.0 M or less. By using the phosphate buffer solution containing the other salt with the above concentration, the separation of the protein into the target fraction can be carried out with excellent accuracy.

When a phosphate buffer solution is used as the eluent, its pH is not particularly limited, but is set to preferably about 5.5 to 8.5, and more preferably about 6.0 to 8.0, thereby reliably eluting the protein, resulting in improvement in the purification rate thereof.

The temperature of the phosphate buffer solution is also not particularly limited, but is preferably about 4 to 50°C, and more preferably about 10 to 35°C, thereby preventing the deterioration (denaturation) of the protein to be separated.

Accordingly, with the phosphate buffer solution having the above pH range and temperature range, the recovery rate of the target protein can be improved.

The phosphate concentration of the phosphate buffer solution is preferably about 0.5 mM or more and 400 mM or less, and more preferably about 1 mM or more and 300 mM or less. By carrying out the separation of the protein using the phosphate buffer solution with the above phosphate concentration, it is possible to prevent metal ions in the phosphate buffer solution from adversely affecting the protein.

Specifically, the phosphate buffer solution having the phosphate concentration of about 1 mM or more and 400 mM or less, and the other salt concentration of about 0.1 M or more and 1.5 M or less, is preferably used. In addition, the phosphate concentration of the phosphate buffer solution is preferably changed continuously or stepwise in the separation of the protein, thereby improving efficiency in the separation of the protein.

The flow rate of the phosphate buffer solution is preferably about 0.1 mL/min or more and 10 mL/min or less, and more preferably about 0.2 mL/min or more and 5 mL/min or less. In the above flow rates, the target protein can be reliably separated, that is, high-purity protein can be obtained, without a long-time separation work.

According the above operations, the protein be separated (isolated) is collected in the predetermined fraction, and then purified.

When repeating the separation of the protein using the adsorption apparatus 1 as described above, the adsorption apparatus 1 is used again for the separation of the protein in the steps [S1A] to [S4A] after washing and equilibrating the adsorbents 3 in steps [S5A] to [S7A] as described below. The steps [S5A] to [S7A] for washing and equilibrating the adsorbents 3 will be described below.

### [S5A] Washing step

As a washing liquid (first washing liquid) for washing the adsorbents 3 by eluting the protein, the foreign protein, etc. which are retained (adsorbed) on the adsorbent 3, for example, a buffer solution is supplied into the column 2 through the inlet pipe 24.

That is, the residues of the protein, the foreign protein, etc., which have not been eluted in the step [S4A], are on the adsorbents 3. Therefore, the adsorbents 3 are washed in the step [S5A] by supplying the washing liquid so as to elute the residues in the washing liquid.

The buffer solution used as the washing liquid is preferably a phosphate buffer solution. The phosphate buffer solution may be the same one as that used as the eluent in the step [S4A].

When the phosphate buffer solution is used as the washing liquid, the phosphate concentration of the phosphate buffer solution is preferably about 200 mM or more and 700 mM or less, and more preferably about 300 mM or more and 500 mM or less. By using the phosphate buffer solution with the above phosphate concentration as the washing liquid, the residues of the protein, the foreign protein, etc., which are adsorbed on the adsorbents 3, can be reliably eluted in the washing liquid.

### [S6A] Alkali-washing step

As a washing liquid (second washing liquid) for further washing the adsorbents 3 by eluting the residues of the protein, the foreign protein, etc. on the adsorbent 3, which have not been eluted even in the step [S5A], for example, an alkali solution is supplied into the column 2 through the inlet pipe 24.

That is, the adsorbents 3 are washed by eluting the residues of the protein, the foreign protein, etc. adsorbed on the adsorbent 3, which have not been eluted by washing with the buffer solution in the step [S5A], in the washing liquid in the step [S6A].

The alkali solution used as the washing liquid may be, although not particularly limited, for example, an aqueous solution containing alkali such as sodium hydroxide, sodium carbonate, potassium hydroxide, potassium carbonate, ammonia.

The alkali concentration of the alkali solution is preferably about 0.1 M or more and 1.5 M or less, and more preferably about 0.5 M or more and 1.0 M or less. By using the alkali solution having the above alkali concentration as the washing liquid, the residues of the protein, the foreign protein, etc. adsorbed on the adsorbents 3 can be reliably eluted in the washing liquid.

### [S7A] pH equilibration step

As an equilibration liquid for equilibrating the pH of the washed adsorbents 3, for example, a buffer solution is supplied into the column 2 through the inlet pipe 24.

That is, because the pH of the adsorbents 3 shows alkalinity due to washing with the alkali solution in the step [S6A], the equilibration liquid is supplied in this step [S7A] to equilibrate the pH of the adsorbents 3 to be near neutral.

The buffer solution used as the equilibration liquid is preferably a phosphate buffer solution. The phosphate buffer solution may be the same one as that used as an eluent in the step [S4A].

When the phosphate buffer solution is used as the equilibration liquid, the phosphate concentration of the phosphate buffer solution is preferably about 200 mM or more and 700 mM or less, and more preferably about 300 mM or more and 500 mM or less. By using the phosphate buffer solution with the above phosphate concentration as the equilibration liquid, the pH of the adsorbents 3 can be reliably equilibrated to near neutral.

Because the adsorbents 3 are washed and equilibrated in the above steps [S5A] to [S7A], the adsorption apparatus 1 can be used for the separation of the protein again.

When a treatment method described below is carried out, the steps [S6A] and [S7A], or the step [S7A], may be omitted among the steps [S5A] to [S7A] in which the adsorbents 3 are washed and equilibrated.

A Ca supplementation step may be carried out before the step [S5A], which supplies a calcium solution into the column 2 through the inlet pipe 24 to supplement the calcium sites of the adsorbents 3 with calcium. The Ca supplementation step [S8A] will be described below.

### [S8A] Ca supplementation step

Before the step [S5A], as the calcium solution for supplementing the calcium sites of the adsorbents 3 with calcium, for example, a calcium-containing buffer solution is supplied into the column 2 through the inlet pipe 24.

That is, after the purification of the protein in the step [S1A] to [S4A], calcium may be detached from some calcium sites of the adsorbents 3 (secondary apatite particles). Therefore, in the step [S8A], the calcium-containing buffer solution is supplied to bring the calcium-lacking sites into contact with calcium included in the buffer solution, resulting in supplementation of calcium.

The buffer solution used as the calcium-containing buffer solution may be, although not particularly limited, the same one as that used as the eluent in the step [S4A], and MES is preferable among them.

Further, the calcium-containing buffer solution is preferably obtained by adding the buffer solution with either or both of a calcium base and a calcium salt. The calcium base and calcium salt may be, for example, calcium hydroxide, calcium carbonate, calcium chloride, calcium nitrate, calcium acetate, calcium lactate, and the hydrate thereof, respectively.

The calcium content of the calcium-containing buffer solution is preferably about 1 mM or more and 100 mM or less, and more preferably about 5 mM or more and 70 mM or less.

The above calcium-containing buffer solution can reliably supplement the calcium-lacking sites with calcium.

### <Treatment method>

The separation of the protein using the adsorption apparatus 1 can be carried out multiple times by repeating the steps [S1A] to [S8A].

However, when repeating the separation of the protein using the adsorption apparatus 1 as described above, high pressure is applied to the adsorbents 3, particularly those positioned near the outlet-side opening in the column body 21, into which a liquid such as the sample liquid is supplied. This turns a particulate shape of the adsorbents 3 (powder) to a sand shape. As the number of the repetition increases, higher pressure is needed to supply the liquid into the column body 21. As a result, the liquid cannot finally be supplied into the column body 21.

The treatment method of the present invention comprises preparing the above adsorbents 3 (powder including secondary particles of apatite) which have been repeatedly used for the separation of the protein, and then bringing the adsorbents 3 into contact with the treatment liquid containing alcohol, or bringing the adsorbents 3 into contact with the treatment liquid containing NaOH for 12 hours or more. Due to the above contact of the adsorbents 3 with the treatment liquid, even when high pressure is applied to the adsorbents 3 positioned near the outlet-side opening in the column body 21 for supplying the liquid such as the sample liquid into the column body 21 in the following separation of the protein using the adsorption apparatus 1, it can be properly suppressed or prevented that the particulate shape of the adsorbents 3 (powder) is turned to a sand shape. Therefore, it can be properly suppressed or prevented that the pressure for supplying the liquid into the column body 21 increases in each separation of the protein, and that the liquid cannot finally be supplied into the column body 21.

Although pressure is also applied to the adsorbents 3, particularly those positioned near the outlet-side opening in the column body 21, for supplying the treatment liquid into the column body 21 in the treatment method of the present invention, when using the treatment liquid containing alcohol or NaOH, it can be properly suppressed or prevented that the particulate shape of the adsorbents 3 (powder) is turned to a sand shape.

FIG. 2 is a flow chart showing the treatment method of the hydroxyapatite filler according to the present invention.

The treatment method of the present invention is applied to the adsorbents 3 (powder including secondary particles of apatite) to be repeatedly used for the separation of the protein, and comprises a step [S1B] of preparing the adsorbents 3 (powder including secondary particles of apatite) that have been used for the separation of the protein, which may be referred to as "used powder" hereinafter, and a step [S2B] of bringing the used powder into contact with the treatment liquid containing alcohol or NaOH.

These steps will be described below.

### [S1B] Preparation step

The adsorbents 3 which have been used for the separation of the protein (the used powder) are prepared.

In this embodiment, the used powder is prepared by the separation method of the protein using the adsorption apparatus 1 as described above, that is, by conducting the steps [S1A] to [S8A].

In other words, the separation method of the protein as described above (the steps [S1A] to [S8A]) constitutes the first step where the first liquid containing a predetermined material is brought into contact with the hydroxyapatite filler.

Also, the separation method of the protein as described above (the steps [S1A] to [S8A]) constitutes the step of preparing the hydroxyapatite filler which has already been used once or more in the step of separating the target material from the liquid composition containing the target material.

In the first step (steps [S1A] to [S8A]), the used powder is brought into contact with a phosphate buffer solution containing phosphate as a predetermined material, as a first liquid in the step [S3A] (supply step), the used powder material is brought into contact with a phosphate buffer solution containing phosphate and other salt as a predetermined, as a first liquid in the step [S4A] [fractionation step (collection step)], the used powder is brought into contact with a phosphate buffer solution containing phosphate as a predetermined material, as a first liquid in the step [S5A] (washing step), the used powder is brought into contact with an alkali solution containing alkali as a predetermined material, as a first liquid in the step [S6A] (alkali-washing step), the used powder is brought into contact with a phosphate buffer solution containing phosphate as a predetermined material, as a first liquid in the step [S7A] (pH equilibration step), and the used powder is brought into contact with a calcium-containing buffer solution containing calcium ion as a predetermined material, as a first liquid in the step [S8A] (Ca supplementation step).

### [S2B] Treatment step

The used powder (adsorbents 3) is brought into contact with the treatment liquid containing alcohol or NaOH.

The treatment step includes the case in which treatment liquid containing alcohol is used, and the case in which treatment liquid containing NaOH is used, as described above.

Thus, the case in which the treatment liquid containing alcohol is used, and the case in which the treatment liquid containing NaOH is used, will be individually described below.

### [S2B-1] Case in which treatment liquid containing alcohol is used

When the treatment liquid containing alcohol is used in the second step, the used powder (adsorbents 3) is brought into contact with the treatment liquid (second liquid) containing alcohol.

By bringing the used powder into contact with the treatment liquid containing alcohol as described above, even when high pressure is applied to the adsorbents 3 positioned near the outlet-side opening in the column body 21 for supplying the liquid such as the sample liquid into the column body 21 in the following separation of the protein using the adsorption apparatus 1 (the steps [S1A] to [S8A]), it can be properly suppressed or prevented that the particulate shape of the adsorbents 3 (powder) is turned to a sand shape. Therefore, it can be properly suppressed or prevented that the pressure for supplying the liquid into the column body 21 increases in each separation of the protein, and that the liquid cannot be supplied into the column body 21. In other words, it is possible to provide the used powder with excellent durability. As a result, the separation of the protein in the sample liquid using the used powder (adsorbents 3) can be stably carried out multiple times.

The alcohol contained in the treatment liquid is preferably, for example, a lower alcohol such as methanol, ethanol, propanol, butanol, pentanol, hexanol, etc., and may be the combination of two or more kinds of the lower alcohol, particularly ethanol is preferable.

The alcohol content of the treatment liquid, which is an aqueous solution containing alcohol in this embodiment, is preferably 20% by weight or more, and more preferably about 20% by weight or more and 35% by weight or less.

Further, the treatment liquid preferably contains calcium ion other than alcohol. The treatment liquid containing calcium ion can be obtained, for example, by adding the aqueous solution containing alcohol with a least one kind of a calcium base and a calcium salt.

The calcium base and calcium salt may include, for example, calcium hydroxide, calcium carbonate, calcium chloride, calcium nitrate, calcium acetate, calcium lactate, and the hydrate thereof, respectively.

The calcium ion concentration of the treatment liquid is preferably about 1.0 mM or more and 20.0 mM or less, and more preferably about 5.0 mM or more and 15.0 mM or less.

By using the above treatment liquid, the hydroxyapatite filler which has been used for the separation of the protein can be reliably provided with excellent durability, so that the separation of the protein can be carried out multiple times.

The time of keeping the adsorbents 3 in the treatment liquid, that is, the time of keeping the adsorbents 3 in contact with the treatment liquid, is preferably 24 hours or more, and more preferably 48 hours or more. On the other hand, it is preferably about 300 days or less.

By setting the time of keeping the adsorbents 3 in contact with the treatment liquid to the above range, it is possible to reliably provide the adsorbents 3 (hydroxyapatite filler) which have been used for the separation of the protein with excellent durability, so that the separation of the protein can be carried out multiple times.

### [S2B-2] Case in which treatment liquid containing NaOH is used

When the treatment liquid containing NaOH is used in the second step, the used powder (adsorbents 3) is brought into contact with the treatment liquid (second liquid) containing NaOH of 0.05 M or less for 12 hours or more.

By bringing the used powder into contact with the treatment liquid containing NaOH of 0.05 M or less for 12 hours or more as described above, even when high pressure is applied to the adsorbents 3 positioned near the outlet-side opening in the column body 21 for supplying the liquid such as the sample liquid into the column body 21 in the following separation of the protein using the adsorption apparatus 1 (the steps [S1A] to [S8A]), it can also be properly suppressed or prevented that the particulate shape of the adsorbents 3 (powder) is turned to a sand shape. Therefore, it can be properly suppressed or prevented that the pressure for supplying the liquid into the column body 21 increases in each separation of the protein, and that the liquid cannot be supplied into the column body 21. In other words, it is possible to provide the used powder with excellent durability. As a result, the separation of the protein in the sample liquid using the adsorbents 3 composed of the used powder can be stably carried out multiple times.

The NaOH content of the treatment liquid, which is an aqueous solution containing NaOH in this embodiment, is 0.05 M or less, and particularly preferably about 0.0 M or more and 0.01M or less.

By using the treatment liquid containing low-concentration NaOH as described above, the hydroxyapatite filler which has been used for the separation of the protein can be reliably provided with excellent durability, so that the separation of the protein can be carried out multiple times.

The time of keeping the adsorbents 3 in the treatment liquid, that is, the time of keeping the adsorbents 3 in contact with the treatment liquid, is 12 hours or more, preferably about 48 hours or more and 30 days or less, and more preferably about 5 days or more and 10 days or less.

By setting the time of keeping the adsorbents 3 in contact with the treatment liquid to the above range, it is possible to reliably provide the adsorbents 3 (hydroxyapatite filler) which have been used for the separation of the protein with excellent durability, so that the separation of the protein can be carried out multiple times.

It is noted that the step [S2B-1] and the step [S2B-2] can be carried out together, and the step [S2B-2] can be carried out after the step [S2B-1].

The method of bringing the adsorbents 3 in contact with the treatment liquid containing alcohol or NaOH, that is, the method of conducting the step [S2B-1] or [S2B-2], is not particularly limited, but preferably includes, for example, a step of passing the treatment liquid through the adsorbents 3 (used powder) in the column body 21 in an amount two times or more as much as that of the adsorbents 3, and then keeping the adsorbents 3 in the treatment liquid. This can stably bring the treatment liquid into contact with the adsorbents 3.

Specifically, the treatment liquid of an amount two times or more as much as that of the adsorbents 3 is supplied into the column body 21 through the inlet pipe 24 for the adsorbents 3 filled in the column body 21 of the column 2. Then, the equilibration liquid retained in the column body 21, which has been supplied in the step [6A], is eluted through the outlet pipe 25, and the supply of the treatment liquid through the inlet pipe 24 is stopped at the time when the equilibration liquid in the column 2 is fully substituted with the treatment liquid. When the supply of the treatment liquid into the column 2 is stopped as described above, the adsorbents 3 are kept in the treatment liquid.

Although pressure is also applied to the adsorbents 3, particularly those positioned near the outlet-side opening in the column body 21, for supplying the treatment liquid into the column body 21, when using the treatment liquid containing alcohol or NaOH as in the step [S2B-1] or [S2B-2], it can be properly suppressed or prevented that the particulate shape of the adsorbents 3 (powder) is turned to a sand shape.

Herein, keeping the adsorbents 3 in the treatment liquid means immersing the adsorbents 3 in the treatment liquid while stopping the supply of the treatment liquid through the inlet pipe 24 and the discharge of the treatment liquid through the outlet pipe 25, which may be conducted, for example, with vibration by ultrasonic wave or a shaker.

Although the adsorbents 3 are kept in the treatment liquid in the step [S2B] described above, the adsorbents 3 have only to be in contact with the treatment liquid, and thus may be treated by continuing the supply of the treatment liquid into the column 2 through the inlet pipe 24 so that the treatment liquid is kept flowing through the adsorbents 3. However, by selecting the method of keeping the adsorbents 3 in the treatment liquid, the waste of the treatment liquid can be reduced. On the other hand, when selecting the method of flowing the treatment liquid through the adsorbents 3, the flow rate of the treatment liquid is preferably set to about 0.1 mL/min or more and 10 mL/min or less, and more preferably about 0.2 mL/min or more and 5 mL/min or less.

A restoration step of obtaining the hydroxyapatite filler (adsorbents 3) with enhanced durability is configured by the treatment of the adsorbents 3 (hydroxyapatite filler) with the treatment liquid containing alcohol or NaOH as described above, that is, the step [S2B].

Because the hydroxyapatite filler (adsorbents 3) which has been used for the separation of the protein can be provided with excellent durability by the above operations, the adsorption apparatus 1 containing these adsorbents 3 can be used for the separation method.

Although protein is used as an example of the charged material in the above description, the negatively charged material may be an acidic amino acid, DNA, RNA, a negatively charged liposome, etc., other than acidic protein. The positively charged material may be a basic amino acid, a positively charged cholesterol, a positively charged liposome, etc., other than basic protein.

The treatment method, the production method and the hydroxyapatite filler of the present invention are described above, however, the present invention is not restricted thereto.

For example, the treatment method and the production method of the present invention may be added with one or more steps for arbitrary purposes.

### EXAMPLE

The specific examples of the present invention will be described below.

### 1. Manufacture of adsorption apparatus

The adsorption apparatus was manufactured by filling the filling space of the stainless steel column (inner diameter 4.0 mm × length 100 mm, filter 2.0 µm) almost fully with hydroxyapatite (CHT 40 µm Type I available from Bio-Rad Laboratories, Inc.) as the adsorbents (hydroxyapatite filler).

### 2. Contact of first liquid and second liquid with adsorbents in adsorption apparatus

### (Example 1)

<1A> The adsorption apparatus was mounted on a chromatograph. Then, 400 mM sodium phosphate buffer solution (pH 6.5, temperature 25°C) flowed through the adsorption apparatus at a flow rate of 0.5 mL/min and a flow volume of 10 CV to substitute the adsorption apparatus with 400 mM sodium phosphate buffer solution.

<2A> 10 mM sodium phosphate buffer solution (pH 6.5, temperature 25°C) flowed through the adsorption apparatus at a flow rate of 0.5 mL/min and a flow volume of 15 CV to equilibrate the adsorption apparatus.

<3A> Sodium phosphate buffer solution (pH 6.5, temperature 25°C) containing 10 mM sodium phosphate and 1.0 M sodium chloride flowed through the adsorption apparatus at a flow rate of 0.5 mL/min and a flow volume of 10 CV to assume the separation step of the protein adsorbed on the adsorbents in the adsorption apparatus.

<4A> 400 mM sodium phosphate buffer solution (pH 6.5, temperature 25°C) flowed through the adsorption apparatus at a flow rate of 0.5 mL/min and a flow volume of 10 CV to wash the adsorption apparatus.

<5A> 1.0 M sodium hydroxide aqueous solution (temperature 25°C) flowed through the adsorption apparatus at a flow rate of 0.5 mL/min and a flow volume of 10 CV to alkali-wash the adsorption apparatus.

<6A> 20% by weight ethanol aqueous solution (temperature 25°C) flowed through the adsorption apparatus at a flow rate of 0.5 mL/min and a flow volume of 10 CV to immerse the adsorbents in the 20% by weight ethanol aqueous solution (second liquid). The filler was preserved for 48 hours in the state where the 20% by weight ethanol aqueous solution (second liquid) was in contact with the adsorbents.

In Example 1, the steps <1A> to <5A> constitute the first step where the first liquid containing a predetermined material is brought into contact with the adsorbents in the adsorption apparatus, and the step <6A> constitutes the second step where the second liquid containing an alcohol is brought into contact with the adsorbents in the adsorption apparatus.

### (Example 2, Comparative Examples 1 to 4)

The first liquid and the second liquid were brought into contact with the adsorbents in the adsorption apparatus in the same manner as in Example 1 except that as the second liquid in the step <6A>, those shown in Table 1 were used in place of 20% by weight ethanol aqueous solution.

### (Examples 3 to 7, Comparative Examples 5 to 9)

The first liquid and the second liquid were brought into contact with the adsorbents in the adsorption apparatus in the same manner as in Example 1 except that as the second liquid in the step <6A>, those shown in Table 1 were used in place of 20% by weight ethanol aqueous solution, and that in the step <6A>, the filler was immersed in the second liquid and preserved for 7 days.

### (Example 8)

The first liquid and the second liquid were brought into contact with the adsorbents in the adsorption apparatus in the same manner as in Example 3 except that a step <7A> below was added before the step <4A>.

<7A> Sodium phosphate buffer solution (pH 6.5, temperature 25°C) containing 5 mM sodium phosphate and 0.5 M sodium chloride flowed through the adsorption apparatus at a flow rate of 0.5 mL/min and a flow volume of 5 CV, MES buffer solution (pH 6.5, temperature 25°C) containing 10 mM calcium chloride and 50 mM MES flowed through the adsorption apparatus at a flow rate of 0.5 mL/min and a flow volume of 5 CV, and then sodium phosphate buffer solution (pH 6.5, temperature 25°C) containing 5 mM sodium phosphate and 0.5 M sodium chloride flowed through the adsorption apparatus at a flow rate of 0.5 mL/min and a flow volume of 5 CV, to supplement adsorbents in the adsorption apparatus with calcium.

### (Reference Example 1)

Only the first liquid was brought into contact with the adsorbents in the adsorption apparatus in the same manner as in Example 1 except that the step <6A> where the second liquid was brought into contact with the adsorbents was omitted.

### (Reference Example 2)

Only the first liquid was brought into contact with the adsorbents in the adsorption apparatus in the same manner as in Example 8 except that the step <6A> where the second liquid was brought into contact with the adsorbents was omitted.

### 3. Evaluation

In each of Examples, Comparative Examples and Reference Examples, the steps <1A> to <7A> where the first liquid and the second liquid were brought into contact with the adsorbents in the adsorption apparatus were repeated up to the point of time when the internal pressure of the adsorption apparatus exceeded the withstand pressure of the column.

The number of the repetition of the steps <1A> to <7A> in each of Examples, Comparative Examples and Reference Examples is shown in Table 1.

It is noted that in Example 3 and Reference Example 1, because the measurement of the number was conducted three times, the average of the measured numbers is shown in Table 1.

**[Table 1]**

| | Step <6A> | | Step <7A> | Repetition Number |
|---|---|---|---|---|
| | Second Liquid (Preservation Liquid) | Preservation Time | Presence: Yes Absence: No | [Cycle] |
| Example 1 | 20 wt% EtOH | 48 hours | No | 43 |
| Example 2 | 20 wt% EtOH + 10 mH Ca + 50 mM MES | 48 hours | No | 42 |
| Example 3 | 20 wt% EtOH | 7 days | No | 48.3 |
| Example 4 | 20 wt% EtOH + 10 mH Ca + 50 mM MES | 7 days | No | 59 |
| Example 5 | 0.05 M NaOH | 7 days | No | 38 |
| Example 6 | 0.01 M NaOH | 7 days | No | 41 |
| Example 7 | Water | 7 days | No | 39 |
| Example 8 | 20 wt% EtOH | 7 days | Yes | 91 |
| Ref. Ex. 1 | - | - | No | 46.7 |
| Ref. Ex. 2 | - | - | Yes | 88 |
| Com. Ex. 1 | 0.1 M NaOH | 48 hours | No | 40 |
| Com. Ex. 2 | 0.1 MNaOH + 10 mM sodium phosphate | 48 hours | No | 31 |
| Com. Ex. 3 | 0.1 MNaOH + 50 mM sodium phosphate | 48 hours | No | 32 |
| Com. Ex. 4 | 0.5 M NaOH | 48 hours | No | 29 |
| Com. Ex. 5 | 0.1 M NaOH | 7 days | No | 29 |
| Com. Ex. 6 | 0.1 MNaOH + 10 mM sodium phosphate | 7 days | No | 27 |
| Com. Ex. 7 | 0.1 M NaOH + 50 mM sodium phosphate | 7 days | No | 26 |
| Com. Ex. 8 | 0.5 M NaOH | 7 days | No | 26 |
| Com. Ex. 9 | 0.1 M KOH | 7 days | No | 32 |

As is clear from Table 1, Examples 5 to 7 where the second liquid (preservation liquid) containing 0.05 M or less of NaOH was used do not exhibit the increasing tendency of the repetition number as compared with Reference Example 1 where the treatment (preservation) in the second liquid (preservation liquid) was omitted, but considering the preservation (contact) time in the second liquid (preservation liquid), exhibit the increasing tendency of the repetition number as compared with Comparative Examples 1 to 8 where the second liquid (preservation liquid) containing more than 0.05 M of NaOH was used, resulting in improvement in the durability of the adsorbents (hydroxyapatite filler).

Examples 1 to 4 and 8 where the treatment (preservation) was conducted using the second liquid (preservation liquid) containing alcohol (ethanol) exhibit the increasing tendency of the repetition number as compared with Comparative Examples 1 to 8 where the second liquid (preservation liquid) containing more than 0.05 M of NaOH was used, and Examples 5 to 7 where the second liquid (preservation liquid) containing 0.05 M or less of NaOH was used, and considering the preservation (contact) time in the second liquid (preservation liquid), unexpectedly as compared with Reference Examples 1 and 2 where the treatment (preservation) in the second liquid (preservation liquid) was omitted, resulting in improvement in the durability of the adsorbents (hydroxyapatite filler).

### DESCRIPTION OF REFERENCE NUMERALS

- 1:: Adsorption apparatus
- 2:: Column
- 3:: Adsorbent
- 4:: Filter member
- 5:: Filter member
- 20:: Adsorbent filling space
- 21:: Column body
- 22:: Cap
- 23:: Cap
- 24:: Inlet pipe
- 25:: Outlet pipe

## Claims

1. A treatment method comprising a first step of bringing a first liquid containing a predetermined material into contact with hydroxyapatite filler, and
a second step of bringing a second liquid containing an alcohol into contact with the hydroxyapatite filler.

2. The treatment method according to claim 1, wherein the alcohol is a lower alcohol.

3. The treatment method according to claim 1 or 2, wherein the content of the alcohol in the second liquid is 20 % or more by weight.

4. The treatment method according to any one of claims 1 to 3, wherein the second liquid further contains a calcium ion.

5. The treatment method according to claim 4, wherein the concentration of the calcium ion in the second liquid is 1.0 mM or more and 20.0 mM or less.

6. A treatment method comprising a first step of bringing a first liquid containing a predetermined material into contact with hydroxyapatite filler, and
a second step of bringing a second liquid containing NaOH with a concentration of 0.05M or less into contact with the hydroxyapatite filler for 12 hours or more.

7. The treatment method according to any one of claims 1 to 6, wherein the second step, in which the second liquid is brought into contact with the hydroxyapatite filler, comprises a process of passing the second liquid through the hydroxyapatite filler in an amount two times or more as much as that of the latter, and then keeping the hydroxyapatite filler in the second liquid.

8. The treatment method according to any one of claims 1 to 7, wherein the first step comprises a process of supplying a phosphate buffer solution containing phosphate as a predetermined material, as a first liquid, into the hydroxyapatite filler,
a recovery process comprising bringing a phosphate buffer solution containing phosphate and other salt as a predetermined material, as a first liquid, into contact with the hydroxyapatite filler,
a process of washing the hydroxyapatite filler by bringing a phosphate buffer solution containing phosphate as a predetermined material, as a first liquid, into contact with the hydroxyapatite filler,
an alkali-washing process comprising bringing an alkali solution containing alkali as a predetermined material, as a first liquid, into contact with the hydroxyapatite filler, and
a pH equilibration process comprising bringing a phosphate buffer solution containing phosphate as a predetermined material, as a first liquid, into contact with the hydroxyapatite filler.

9. The treatment method according to any one of claims 1 to 8, wherein the first step further comprises a process of supplementing Ca by bringing the hydroxyapatite filler into contact with the first liquid containing a calcium ion, before the washing process.

10. A production method comprising the steps of treating the hydroxyapatite filler by the treatment method according to any one of claims 1 to 9,
preparing a liquid composition containing a target material, and
separating the target material from the liquid composition using the hydroxyapatite filler treated in the treatment step.

11. A production method comprising a step of preparing the hydroxyapatite filler which has already been used once or more in a step of separating a target material from a liquid composition containing it, and
a restoration step of providing the hydroxyapatite filler with improved durability by treating the hydroxyapatite filler by the treatment method according to any one of claims 1 to 9.

12. Hydroxyapatite filler obtained by the production method according to claim 11.
